# EUROPEAN PATENT SPECIFICATION

(11) **EP 0 548 237 B1**
(45) Date of publication and mention of the grant of the patent: **09.08.1995**
(21) Application number: 91917122.3
(22) Date of filing: 10.09.1991
(51) Int. Cl.: C08G 63/08, C08G 63/80, A61L 27/00, A61L 31/00

(54) **Method for the production of an article from the Copolymer of Lactide and (e)-Caprolactone for medical applications**
Verfahren zur Herstellung eines Artikels für medizinische Anwendungen aus Copolymeren aus Lactid und E-Caprolacton
Procédé de production d'un article à usage médical à partir d'un Copolymère de Lactide et de Caprolactone-(e)

(30) Priority: 10.09.1990 NL 9001984
(43) Date of publication of application: 30.06.1993
(73) Proprietor: Rijksuniversiteit te Groningen, 9712 CP Groningen (NL)
(72) Inventor: GRIJPMA, Dirk, Wybe, NL-9722 BT Groningen (NL); PENNINGS, Albert, Johan, NL-9331 BE Norg (NL)
(74) Representative: Smulders, Theodorus A.H.J., Ir.
(86) International application number: NL9100164
(87) International publication number: WO9204393

(56) References cited:
- EP-A- 0 108 635
- WO-A-84/04311
- US-A- 4 045 418
- US-A- 4 057 537
- Polymer Bulletin, vol. 25, no. 3, March 1991, Springer Verlag (Berlin, DE) D.W. Grijpma et al.: "Polymerization temperature effects on the properties of L-lactide and epsilon-caprolactone copolymers", pages 335-341, see pages 335-338

## Description

The invention relates to a method for the production of an article from a copolymer of lactide and ε-caprolactone for medical applications.

### Background of the Invention

A method of this type is disclosed in JP-58-122643. In this publication it is described that films and sheets which can be used as anti-adhesive material can be made from a lactide/ε-caprolactone copolymer. During an operation, the sheet or film is inserted between organs or tissues which must not grow together post-operatively. To this end, copolymers are synthesized from monomers which are polymerized in a weight ratio of 25 to 75 parts of lactide to 75 to 25 parts of ε-caprolactone in the presence of tin octoate at a temperature of 160°C.

In surgery there is a need in certain applications for materials which are biocompatible and biodegradable, without toxic substances being released during degradation, but where the materials also have a high tensile strength and elasticity. The material as described in JP-A-58-122643 is unsuitable for a number of applications because this material does not have the desired tensile strength and elasticity.

In US 4,057,537 and in WO-A-84/03 411 it is described how copolymers of purified L-(-)-lactide and purified ε-caprolactone are synthesized at a temperature above the melting point of the lactide. According to US 4,057,537, a lower polymerization temperature can be chosen, but then only in a suspension or solution in an inert medium. This lower temperature is regarded as unfavourable and is alleged to yield less desired polymers.

### Summary of the Invention

The aim of the invention is to provide a method which yields a copolymer which has a higher tensile strength than was known in the prior art and which is suitable for the production of materials for flexible implants such as meniscus, artificial skin or an artificial blood vessel.

According to the invention this is achieved in that the copolymer is obtained by a synthesis which is carried out as a bulk synthesis at a temperature of 80°C to a temperature below the melting point of lactide

More specifically, this invention is a method for the production of an article from the copolymer of lactide and ε-caprolactone for medical applications, comprising:
- purifying the lactide and the ε-caprolactone,
- introducing the purified lactide and the purified ε-caprolactone into a vessel and, allowing the purified lactide and purified caprolactone to react by bulk synthesis at a temperature from about 80°C to a temperature below the melting point of lactide to form a copolymer,
- shaping the copolymer into an article.

By this means it is surprisingly possible to obtain a copolymer having a molecular weight of from 500,000 to 1,000,000 which combines a tensile strength of at least 34 MPa with a high elongation at break.

The synthesis is carried out at 80°C to a temperature below the melting point of lactide, that is to say at a temperature below 98°C.

By the term "bulk synthesis" herein is meant a synthesis in which the reaction medium does not contain solvents and which also does not take place in a suspension or emulsion.

An article can be produced from the copolymer in any known way. Known methods comprise injection molding, hot forming, cutting, milling, sawing and also synthesis in a mold having virtually the shape which the article must acquire.

According to the invention, the lactide and the ε-caprolactone can be added in any desired weight ratio and are preferably added in a weight ratio of 10:90 to 99.1:0.1 and more preferentially in a ratio of 45:55 to 55:45.

If copolymers are made with small amounts of ε-caprolactone, for example 2 to 5 mol-%, the result is a material which has a high modulus, tensile strength and impact strength. It can then, for example, be used for the production of bone plates and screws.

In general, the synthesis is carried out in the presence of a catalyst. The catalyst can be chosen from all catalysts suitable for lactones and carbonates, as are known from the technology to those skilled in the art. Examples of such catalysts are the catalysts as described in U.S. 4,539,981, (& EP-A-108 635) hereby incorporated by reference, that is to say tin octoate, antimony trifluoride, zinc powder, dibutyltin oxide and tin oxalate.

A preferred catalyst consists of a compound according to Formula I: 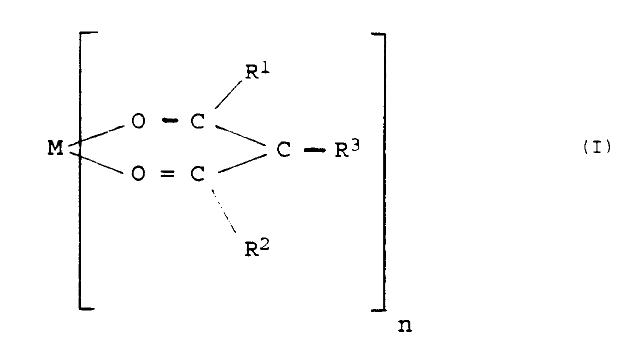
in which M is a metal ion and n is a number from 1 to 4 and equal to the valency of the metal ion and in which the groups R¹ and R² independently of independently of one another are an alkyl, aryl or cycloaliphatic group and R³ is an alkyl, aryl or cycloaliphatic group or a hydrogen atom. It is further possible that the alkyl, aryl or cycloaliphatic groups which form part of R¹, R² or R³ are substituted by halogens.

More preferentially, a catalyst such as described in Formula I is used in which M is a Zn²⁺ and R¹ is a tertiary butyl group and R² is an ethyl group and R³ is an H, in which case the compound has the name bis-(2,2-dimethyl-3,5-heptanedionato-O,O')-zinc, or where M is an Sn²⁺ and R¹ and R² are a methyl and R³ is an H, in which case the compound has the name bis-(2,4-pentanedionato-O,O')-tin(II).

Tin octoate is a catalyst of secondary preference.

The catalyst is present in an amount of 10⁻⁷ to 10⁻³ and preferably approximately 10⁻⁵ mol/mol relative to the monomers.

The synthesis is preferably continued until less than 0.1% by weight of residual monomer remains in the reaction mixture. In general, this will be a period of from 100 to 400 hours. The polymerization time required depends on the polymerization temperature. In addition, the time is dependent on the concentration of the catalyst.

The lactide can be chosen from L-lactide, D-lactide, DL-lactide and mixtures hereof.

The lactide and ε-caprolactone monomers are purified before use to remove contaminants. In the case of lactide, for example, purification is possible by recrystallisation from toluene dried over sodium. ε-Caprolactone can be purified, for example, by distillation under a reduced N₂ atmosphere from CaH₂.

Preferably, the two monomers are purified until their contamination by impurity containing hydroxyl groups is less than 1 ppm.

In a further embodiment of this invention, additional other monomers can be added to the reaction mixture in amounts of from none up to a percentage by weight of 50%. These monomers are preferably chosen from other lactones, such as glycolide, dioxanone, 1,4-dioxane-2,3-dione, β-propiolactone, tetramethylglycolide, β-butyrolactone, γ-butyrolactone or pivalolactone, or cyclic carbonates such as trimethylene carbonate, 2,2-dimethyl-trimethylene carbonate and the like.

The lactones can consist of the optically pure isomers or of two or more optically different isomers.

Furthermore, in another embodiment of this invention, comonomers based on hydroxy-carboxylic acids can be incorporated. Incorporation is possible, for example, amounts of from none a percentage by weight of 50%, but is preferably not higher than about 10%. Said comonomers can be chosen, for example, from the group consisting of
α-hydroxybutyric acid,
α-hydroxyisobutyric acid,
α-hydroxyvaleric acid,
α-hydroxyisovaleric acid,
α-hydroxycaproic acid,
α-hydroxyisocaproic acid,
α-hydroxy-α-ethylbutyric acid,
α-hydroxy-β-methylvaleric acid,
α-hydroxyheptanoic acid,
α-hydroxyoctanoic acid,
α-hydroxydecanoic acid,
α-hydroxymyristic acid,
α-hydroxystearic acid or combinations thereof.

The resulting material can be reinforced by mixing fibrous material into the reaction mixture or by combining the resulting material, by melting or otherwise, with fibrous reinforcing material. The fibrous material can be chosen from all possible biocompatible fibers and is preferably chosen from the highly oriented, strong and rigid fibers. The fibers may or may not be biodegradable. Examples are polylactide fibers, polyglycolide fibers, fibers of lactide/glycolide copolymers and fibers of copolymers of lactide and trimethylene carbonate. The fibrous material can be present in the form of loose fibers, mats, woven fabrics, knitted fabrics or otherwise.

The conventional fillers, inhibitors, release agents, etc. can also be added to the mixture.

Articles prepared according to the invention have numerous uses, but are in particular advantageous if they are employed in biomedical fields. Examples are an implant for the repair of a meniscus or a blood vessel, stents or artificial skin. Stents are small spirals which are used to hold open (coronary) arteries which have deposits on inner walls. Use in abdominal wall reconstruction is also possible.

It is also possible to make nerve guides from the copolymers. A nerve guide is a small tube which can be used to enable a nerve branch which has been broken by a fracture or otherwise to grow together again through the tube, after which the tube degrades.

### Description of Preferred Embodiments

The invention will be illustrated with the aid of the following examples.

The intrinsic viscosity was measured in chloroform at 25°C using an Ubbelohde viscometer.

Monomer conversion and copolymer composition were determined using 'H NMR at 300 MHz in solutions in deuterated chloroform.

The average length of the monomer sequences was determined using '3C NMR at 75 MHz.

Thermal properties were determined using a Perkin Elmer DSC-7, 5-10 mg samples being heated at a rate of 20°C per minute.

The stress-strain behaviour was determined on an Instron 4301 tensile tester, 4 x 50 x 1 mm samples cut from 3 by 10 by 0.1 cm plates obtained by compression molding at room temperature being measured at a crosshead speed of 10 mm/min. The yield strength (that is to say the yield stress), the elongation at break and the ultimate tensile strength were determined from these measurements.

Dynamic thermal analysis was carried out on a Rheometrics RSA-II DMTA. In the tension mode under a constant load of 50 g, the 4 x 50 x 1 mm samples obtained by compression molding and cutting were exposed to an oscillating strain with a maximum amplitude of 0.5% at a frequency of 1 Hz. The heating rate was 5°C/min.

Examples II, III, IV, VI and VIII do not belong to the invention; they are outside the scope of the claims and are given as comparative examples.

### Example I

L-Lactide (CCA, The Netherlands) was purified by recrystallisation from toluene dried over sodium. ε-Caprolactone (Janssen Chemica, Belgium) was purified by drying over CaH₂ and distilling under reduced pressure in a nitrogen atmosphere. The monomers were introduced, in a ratio of 50:50 mol/mol into silanised glass ampoules which had been placed under vaccuum and into which tin octoate (Sigma Corp., USA) was also added in an amount of 1 x 10⁻⁵ mol of catalyst per mol of monomer.

The reaction was carried out for 60 days at a temperature of 80°C.

As a consequence of the difference in reactivity between L-lactide and ε-caprolactone, the lactide first reacted preferentially and only then the ε-caprolactone. This results in copolymers which have a block-like structure and have an average sequence length of 11.0 for the L-lactide and 5.5 for the ε-caprolactone. The intrinsic viscosity was 11.5 dl/g.

### Example II

The method of Example I was followed at a reaction temperature of 110°C. Virtually complete conversion (to a percentage by weight of less than 2% residual monomer) was achieved after 10 days, after which the reaction was stopped. Copolymers having a block structure were formed, which had an average sequence length of 8.5 for the L-lactide and 3.7 for the ε-caprolactone. The intrinsic viscosity was 9.9 dl/g.

Probably the sequence length at 110°C is shorter than at 80°C because of a higher degree of transesterification during the raction. The fact that the difference in reactivity between L-lactide and ε-caprolactone is less pronounced at higher temperatures may also play a role. A longer sequence length is related to a higher crystallinity and thus to a higher rigidity. As a consequence, the polymer material in Example I is more rigid than the polymer material in Example II, while the material in Example II is tougher than the material in Example I.

### Example III

The method of Example I was followed at a reaction temperature of 120°C for 5 days until the residual monomer content was less than 2% by weight. Copolymers having a block structure were formed, which had an average sequence length of 5.5 for the L-lactide and 2.5 for the ε-caprolactone. The intrinsic viscosity was 7.8 dl/g.

### Example IV

The method of Example I was followed at a reaction temperature of 160°C for 2 days until the residual monomer content was less than 2% by weight. Copolymers having a block structure were formed, which had an average sequence length of 3.9 for the L-lactide and 2.1 for the ε-caprolactone. The intrinsic viscosity was 3.4 dl/g.

It can be concluded from the sequence lengths of the blocks in the polymers in Examples I to IV that the chain becomes more block-like when the reaction temperature is lowered. The mechanical properties, and specifically the tensile strength, become better as a result. The mechanical properties of the polymer from Example II are comparable with the properties of, for example, Estane®, a type of polyurethane used in biomedical application.

### Example V

The reaction product from Example I was processed by compression molding at 200°C, followed by slow cooling and annealing for 8 hours at 100°C, to give 3 by 10 by 0.1 cm plates.

### Example VI

The reaction product from Example II was processed in the manner described in Example V to give measuring rods.

### Example VII

Using a DSC scan, a T_{g} of -39°C was determined for the measuring rods from Example V, which indicates an amorphous phase rich in ε-caprolactone. The material also had two melt endotherms: one at 44.3°C with a ΔH of 5.8 J/g and one at 149°C, with a ΔH of 14.8 J/g. This indicates two crystalline phases: a poly-ε-caprolactone phase and a poly-L-lactide phase. No indications for an amorphous L-lactide phase were found.

The stress-strain behaviour of the measuring rods was also measured. The results of these measurements are given in Table 1.

### Example VIII

Using a DSC scan, a T_{g} at -15°C and a T_{g} at 55°C were measured for the measuring rods from Example VI, which indicates two different amorphous phases. The second, which is rich in poly-L-lactide, displays an enthalpy recovery peak. The material had a melt endotherm Tₘ at 102°C with a ΔH of 4.0 J/g, which melt endotherm indicates the melting of small and imperfect L-lactide crystallites. The material was completely amorphous before processing and displayed some crystallinity only after annealing for 3 weeks at room temperature.

The stress-strain behaviour of the measuring rods was also measured. The results are given in Table 1.

**Table 1**

| Results of the stress-strain measurements | | |
|---|---|---|
| | Example VII 80°C | Example VIII (comparison) 110°C |
| Initial modulus (MPa) | 84 | 5.2 |
| Elongation at break (%) | 480 | 880 |
| Tensile strength (MPa) | 5.0 | 1.0 |
| Ultimate tensile strength (MPa) | 18.2 | 9.0 |

It can be concluded from this that a lowering of the reaction temperature leads to copolymers with longer average sequence lengths of the respective comonomers, which leads to higher moduli and tensile strengths, while the elongation at break is lower. The maximum achievable molecular weight is higher.

## Claims

1. A method for the production of an article from the copolymer of lactide and E-caprolactone for medical applications, comprising:
- purifying said lactide and said ε-caprolactone
- introducing said purified lactide and said purified ε-caprolactone into a vessel and
- allowing said purified lactide and said purified ε-caprolactone to react by bulk synthesis at a temperature of from 80°C to a temperature below the melting point of lactide to form said copolymer, then
- shaping said copolymer into said article.

2. The method according to claim 1, wherein the lactide and ε-caprolactone monomers are reacted with one another in a ratio of 10:90 to 99.9:0.1.

3. The method according to claim 1-2, wherein the lactide and ε-caprolactone monomers are reacted with one another in a ratio of 44:55 to 55:45.

4. The method according to claim 1, wherein the monomers are purified before use until they contain an amount of impurities containing containing hydroxyl groups of less than 1 ppm before use.

5. The method according to claim 1, whereby the synthesis is carried out in the presence of a catalyst, wherein the catalyst is chosen from the group consisting of tin octoate, antimony trifluoride, zinc powder, dibutyltin oxide and tin oxalate, bis-(2,2-dimethyl-3,5-heptanedionato-0,0')-zinc and bis-(2,2-pentanedionato-0,0')-tin(II).

6. The method according to claim 5, wherein the catalyst is present in an amount of 10-7 to 10-3 mol/mol relative to the monomers.

7. The method according to claim 1, wherein the synthesis is continued until less than 0.1% by weight of residual monomer remains in the reaction mixture.

8. The method according to claim 1, wherein the copolymer is reinforced with fibers.

## Patentansprüche

1. Verfahren zur Herstellung eines Gegenstands aus einem Copolymer aus Lactid und ε-Caprolacton für medizinische Anwendungszwecke, wobei man:
- das genannte Lactid und das genannte ε-Caprolacton reinigt,
- das genannte gereinigte Lactid und das genannte gereinigte ε-Caprolacton in ein Gefäß überführt und
- das genannte gereinigte Lactid und das genannte gereinigte ε-Caprolacton mittels Masse-Synthese bei einer Temperatur von 80°C bis unterhalb der Schmelzpunkttemperatur des Lactids reagieren läßt, um das genannte Copolymer zu bilden, und dann
- das genannte Copolymer zu dem genannten Gegenstand formt.

2. Verfahren gemäß Anspruch 1, worin die Lactid- und ε-Caprolacton-Monomeren miteinander in einem Verhältnis von 10:90 bis 99,9:0,1 umgesetzt werden.

3. Verfahren gemäß Anspruch 1 bis 2, worin die Lactid- und ε-Caprolacton-Monomeren miteinander in einem Verhältnis von 45:55 bis 55:45 umgesetzt werden.

4. Verfahren gemäß Anspruch 1, worin die Monomeren vor dem Gebrauch gereinigt werden, bis sie eine Menge an Hydroxylgruppen enthaltenden Verunreinigungen von weniger als 1 ppm enthalten.

5. Verfahren gemäß Anspruch 1, wobei die Synthese in der Gegenwart eines Katalysators durchgeführt wird, wobei der Katalysator aus der Gruppe ausgewählt ist, bestehend aus Zinnoktoat, Antimontrifluorid, Zinkpulver, Dibutylzinnoxid und Zinnoxalat, Bis(2,2-dimethyl-3,5-heptandionato-0,0')zink und Bis(2,4-pentandionato-0,0')zinn(II).

6. Verfahren gemäß Anspruch 5, worin der Katalysator in einer Menge von 10⁻⁷ bis 10⁻³ Mol/Mol, bezogen auf die Monomeren, vorhanden ist.

7. Verfahren gemäß Anspruch 1, worin die Synthese weitergeführt wird, bis weniger als 0,1 Gew.% restliches Monomer in der Reaktionsmischung verbleibt.

8. Verfahren gemäß Anspruch 1, worin das Copolymer mit Fasern verstärkt wird.

## Revendications

1. Procédé de production d'un article à partir du copolymère de lactide et d'ε-caprolactone pour des applications médicales , comprenant les étapes consistant à :
- purifier ledit lactide et ladite ε-caprolactone
- introduire ledit lactide purifié et ladite ε-caprolactone purifée dans un récipient et
- laisser réagir le lactide purifié avec ladite ε-caprolactone purifiée par synthèse en masse à une température comprise entre 80°C et une température inférieure au point de fusion du lactide pour former ledit copolymère, puis
- façonner ledit copolymère en ledit article.

2. Procédé selon la revendication 1, dans lequel on fait réagir les monomères de lactide avec ceux d'ε-caprolactone dans un rapport de 10/90 à 99,9/0,1.

3. Procédé selon la revendication 2, dans lequel on fait réagir les monomères de lactide avec ceux d'ε-caprolactone dans un rapport de 44/55 à 55/45.

4. Procédé selon la revendication 1, dans lequel les monomères sont purifiés avant utilisation jusqu'à ce qu'ils contiennent une quantité d'impuretés contenant des groupes hydroxyle inférieure à 1 ppm avant utilisation.

5. Procédé selon la revendication 1, dans lequel la synthèse est mise en oeuvre en présence d'un catalyseur, ce catalyseur étant choisi dans le groupe constitué d'octoate d'étain, de trifluorure d'antimoine, de poudre de zinc, d'oxyde de dibutylétain et d'oxalate d'étain, de bis-(2,2-diméthyl-3,5-heptanedionato-0,0')-zinc et de bis-(2,2-pentanedionato-0,0')-étain(III).

6. Procédé selon la revendication 5, dans lequel le catalyseur est présent en une quantité comprise entre 10/7 et 10/3 mol/mol par rapport aux monomères.

7. Procédé selon la revendication 1, dans lequel on poursuit la synthèse jusqu'à ce qu'il reste moins de 0,1 % de monomère résiduel dans le mélange réactionnel.

8. Procédé selon la revendication 1, dans lequel le copolymère est renforcé par des fibres.
